# EUROPEAN PATENT SPECIFICATION

(11) **EP 1 943 203 B1**
(45) Date of publication and mention of the grant of the patent: **19.12.2012**
(21) Application number: 06836922.2
(22) Date of filing: 03.11.2006
(51) Int. Cl.: C07C 17/25, C07C 21/18, C07C 17/04, C07C 19/10, C07C 19/08

(54) **METHOD FOR PRODUCING FLUORINATED ORGANIC COMPOUNDS**
VERFAHREN ZUR HERSTELLUNG FLUORIERTER ORGANISCHER VERBINDUNGEN
PROCEDE DE PRODUCTION DE COMPOSES ORGANIQUES FLUORES

(30) Priority: 03.11.2005 US 733355 P
(43) Date of publication of application: 16.07.2008
(73) Proprietor: Honeywell International Inc., Morristown, NJ 07960 (US)
(72) Inventor: MUKHOPADHYAY, Sudip, Williamsville, NY 14221 (US); NAIR, Haridasan, Williamsville, NY 14221 (US); VAN DER PUY, Michael, Amherst, NY 14221 (US); TUNG, Hsueh Sung, Getzville, US 14068 (US); MERKEL, Daniel, C., West Seneca, US 14224 (US); DUBEY, Rajesh, Buffalo, NY 14210 (US); MA, Jing Ji, West Seneca, NY 14224 (US)
(74) Representative: Clarke, Lionel Paul
(86) International application number: PCT/US2006/043053
(87) International publication number: WO 2007/056194

(56) References cited:
- EP-A1- 0 644 173
- WO-A-03/027051
- WO-A-2005/042451
- WO-A-2005/108334
- WO-A-2007/019355
- US-A1- 6 031 141
- KNUNYANTS I L ET AL: "REACTIONS OF FLUORO OLEFINS COMMUNICATION 13. CATALYTIC HYDROGENATION OF PERFLUORO OLEFINS" BULLETIN OF THE ACADEMY OF SCIENCES OF THE USSR, DIVISION OF CHEMICAL SCIENCES, 1960, pages 1312-1317, XP000578879 ISSN: 0568-5230
- KUNSHENKO B V ET AL: "REACTION OF ORGANIC COMPOUNDS WITH SF4-HF-HALOGENATING SYSTEM VII. REACTIONS OF OLEFINS WITH THE SF4-HF-CL2(BR2)SYSTEM" JOURNAL OF ORGANIC CHEMISTRY OF THE USSR, XX, XX, vol. 28, 1992, pages 511-518, XP002344564 ISSN: 0022-3271
- DATABASE BEILSTEIN BEILSTEIN INSTITUTE FOR ORGANIC CHEMISTRY, FRANKFURT-MAIN, DE; XP002426121 Database accession no. Reaction ID 224017 & HAZELDINE: J. CHEM. SOC., 1952, page 1504, 2506, & M. BUECHNER ET AL.: CHEM. EUROP. J., vol. 4, no. 9, 1998, pages 1799-1809,
- GAMBARETTO, G. P. ET AL: "The reactions of chlorine monofluoride with unsaturated compounds and the dehydrohalogenation of some of the derivatives" JOURNAL OF FLUORINE CHEMISTRY, vol. 7, no. 6, 1976, pages 569-580, XP002426119

## Description

### BACKGROUND OF INVENTION

### (1) Field of Invention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1, 3,3,3-tetrafluoro-1-propene (HFO-1234ze)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts, including a sizeable amount of carbon black. The carbon black is not only unwanted, it tends to deactivate the catalyst used in the process.

Knunyants, L. et al. (Bulletin of the Academy of Sciences of the USSR, Division of Chemical Sciences, 1960, pages 1312-1317) describe the dehydrofluorination of 1,1,1,2,3-pentafluoropropane to 2,3,3,3- tetrafluoropropene. 1,1,1,2,3-pentafluoropropane (20g) was added with stirring to a suspension of 20g of KOH in dibutyl ether. The reaction products were collected in a trap cooled to -78°C and were then passed again through the suspension of alkali. Distillation of the reaction products through a column gave 11.5 g (70%) of 2,3,3,3-tetrafluoropropene.

The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Notwithstanding prior teachings applicants appreciate a continuing need for methods of efficiently preparing certain hydrofluorocarbons, particularly tetrafluorpropenes such as HFO-1234yfand HFO-1234ze (including cis- and trans-forms thereof).

### SUMMARY OF THE INVENTION

Applicants have developed a method for producing fluorinated organic compounds, including hydrofluoropropenes, which preferably comprises converting a compound of formula (I):

CF₃CHFCH₂F (HFC-245eb) (I)

to a compound of formula (II)

CF₃CF=CF₂ (HFO-1234yf) (II)

The converting step of the present invention comprises dehydrohalogenating at least one compound of formula (I). The dehydrohalogenation step comprises in preferred embodiments introducing said at least one compound of formula (I) to a reaction system under conditions effective to convert, and preferably convert at least 50%, and even more preferably at least 70%, of said compound of formula (I). It is also generally preferred that said reaction step produces a reaction product having at least 70% selectivity, and even more preferably at least 80% selectivity, to compounds of formula (II). In certain highly preferred embodiments, the reaction step produces a reaction product having at least 70% selectivity, and even more preferably at least 80% selectivity, to HFO-1234yf.

In certain preferred embodiments, the converting step comprises reacting a compound of formula (I) in the gas phase, in the liquid phase, or a combination of these, with gas phase reactions preferably occurring in the presence of catalyst.

### DETAILED DESCRIPTION OF PREFERRED EMBODIMENTS

One beneficial aspect of the present invention is that it enables the production of the desirable fluroolefin HFO-1234yf, from the relatively attractive starting material, CF₃CH=CH₂ and in preferred embodiments the present method is capable of achieving very desirable levels of conversion of the starting material while also providing high levels of selectivity to the desired products. Flouropropenes in general, and trifluorpropenes such as CF₃CH=CH₂ in particular are in many embodiments an advantageous starting material because such products are relatively inexpensive, are relatively easy to handle, and are generally readily available in commercial quantities or can be easily produced from other readily available materials. For example trifluorpropene can be synthesized by the Cu-catalyzed liquid-phase coupling of CCl₄ and CH₂=CH₂, preferably followed by hydrofluorination.

Thus, the present method includes the step of reacting a fluorinated olefin, with a halogen addition agent, wherein the halogen addition agent is fluorine gas, under conditions effective to produce a compound of formula (I)

CF₃CHFCH₂F (HFC-245eb) (I)

The fluorinated olefin reactant is a compound of formula (III)

CF₃CH=CH₂ (III)

The reaction by which the compound of formula (III) is converted to a compound of formula (I) is sometimes referred to herein for convenience, but not necessarily by way of limitation, as a halogen addition reaction.

The formula (I) compound, which is formed by a process comprising a halogen addition reaction, is then exposed to reaction conditions, which are sometimes referred to herein for convenience, but not necessarily by way of limitation, as a dehydrohalogenation reaction, to produce a reaction product containing a compound of formula (II). Preferred aspects of each of the preferred steps is described below, with the titles used as headings for these steps being used for convenience but not necessarily by way of limitation.

### I. HALOGEN ADDITION

In preferred embodiments, the reactant compound is CF₃CH=CH_{2.} The halogen addition agent is F₂

In certain preferred embodiments, the halogen addition step comprises contacting, (preferably by introducing into a reactor) the compounds in an F₂ formula (III) mole ratio of from 0.01:1 to 50:1, and even more preferably of from 0.1:1 to 0:1. In preferred embodiments in which the halogen addition agent is F₂ and the formula III compound comprises CF₃CH=CH2, the F_{2:}trifluoropropene mole ratio of the feeds to the reactor are from 0.01:1 to 10:1 and even more preferably from 0.1:1 to 1:1.

This reaction step can be carried out in the liquid phase or in the gas phase, and it is contemplated that the reaction can be carried out batch wise, continuous, or a combination of these.

### A. PREFERRED GAS/LIQUID PHASE REACTIONS

The formation of a compound of formula I, particularly a compound of formula (I) (CF₃CHFCH₂F) may also be carried out at least partially in a liquid phase reaction using F₂ as the halogen addition agent where the F₂ is introduced to the reaction mixture as a gas. For the purpose of convenience, but not by way of limitation, such a reaction arrangement is sometimes referred to herein as a gas/liquid phase reaction. Thus, for certain preferred embodiments, particularly those preferred embodiments which utilize F₂ as a reactant, it is preferred to provide a compound of formula (I) by a reaction which is conducted primarily in the liquid phase but in which the F₂ reactant is introduced in the gas phase. In such embodiments HF is preferably used as a solvent (preferably an inert solvent) for the reaction and a catalyst is not required. In certain of such preferred embodiments the F₂ is provided in diluted form, preferably blended with an inert gas, such as nitrogen, in amount of 5 - 100% (preferably about 10%) of the total of F₂ and inert gas. The gas is preferably contacted with the compound of formula (III), preferably in some cases by bubbling the gas through the liquid in a stirred tank reactor at a temperature of from -20°C to -55°C for a time of from 0.5 to 1.5 hours. Preferred reactor pressure is from 1.03 to 5.52 bar (15 to 80 psia) and even more preferably from 20 to 1.38 to 4.83 bar (20 to 70 psia). In such embodiments the conversion of the formula III compound is preferably at least 80 to 100%, more preferably at least 40 to 60%, and selectivity to compounds of formula (I) is preferably at least 30%, more preferably at least 35%, and even more preferably at least 40%.

### 8. PREFERRED GAS PHASE REACTIONS

For certain preferred embodiments, particularly those preferred embodiments which utilize F₂ as a halogen addition agent, it is preferred to provide a compound of formula (1) by a gas-phase reaction. In such preferred embodiments, the compound of formula (III) and the halogen addition agent are introduced into and appropriate reaction vessel in the form of a gas and the reactor is preferably maintained at a temperature of from about -18°C for a time of from 5 minutes to 16 hours, and the reaction products are produced mainly as liquids which separate from the gaseous reactants in the vessel. Preferred reactor pressure is atmospheric. In such embodiments the conversion of the formula III compound is preferably at least 5%, more preferably at least 10%, and selectivity to compounds of formula (I) is preferably at least 30%, more preferably at least 35%, and even more preferably at least 50%.

### II. FORMATION OF THE COMPOUND OF FORMULA II

The methods of the present invention preferably comprise contacting a compound of formula (I) with a dehydrohalogenation agent to produce a a compound of formula (II).

In certain preferred embodiments, the present dehydrohalogenation step is carried out under conditions effective to provide a formula (I) conversion of at least 40%, more preferably at least 55%, and even more preferably at least 70%. In certain preferred embodiments the conversion is at least 90%, and more preferably 100%. Further in certain preferred embodiments, the conversion of the compound of formula (I) to produce a compound of formula (II) is conducted under conditions effective to provide a formula (II) selectivity of at least 25%, more preferably at least 40%, more preferably at least 70%, and even more preferably at least 90%.

This reaction step can be carried out in the liquid phase or in the gas phase, or in a combination of gas and liquid phases, and it is contemplated that the reaction can be carried out batch wise, continuous, or a combination of these.

### A. LIQUID PHASE DEHYDROHALOGENATION

One preferred reaction step may be described, by way of illustration but not necessarily by way of limitation, by the following reaction equation in connection with embodiments in which the compound of formula (I) is 1,1,1,2,3 pentafluoropropane and the dehydrohalogenating agent is potassium hydroxide (KOH):

**CF₃CHFCH₂F + KOH 〉̶ CF₃CF=CH₂ + KF + H₂O**

In such embodiments the KOH is preferably provided as an aqueous solution comprising from 10% to 50% by weight KOH, more preferably from 20% to 30% by weight.

In certain preferred embodiments, the KOH solution is brought to a relatively cool temperature, preferably from -10°C to 10°C, preferably about 0°C and introduced into a reaction vessel. The appropriate amount of formula (I) compound, which is preferably from 1 to 100 mole%, preferably, 0.9 to 10 mole %, is then added to the reaction vessel. The reaction mixture is gradually heated, preferably with the addition of kinetic energy (agitation or stirring) to from 40°C to 80°C, more preferably form 50°C to 60°C. Since the preferred reaction is exothermic, the temperature of the reaction mixture may be allowed to increase to a temperature of from 60°C to 95°C more preferably form 65°C to 75°C. The reaction pressure in such embodiments may vary, depending on particular processing parameters of each application, but in certain embodiments ranges from 0 to 13.79 bar (0 to 200 psig) during the course of the reaction. In certain embodiments the reaction the exothermic heat of reaction is removed (such as by cooling) from the reaction mixture so as to maintain the reaction temperature in the range first mentioned above. The overall reaction time in certain preferred embodiments is from 5 to 40 hours, more preferably from 10 to 30 hours, and even more preferably for 20 hours.

After the desired reaction time, the reaction mixture is preferably cooled to facilitate collection of the reaction product, for example to 20°C to down to 40°C. Preferably, the conversion, and selectivity to HFO-1234yf, are each at least 90% and more preferably at least 95%.

### B. GAS PHASE DEHYDROHALOGENATION

Thus, it is contemplated that the dehydrohalogenation reaction step may be preformed using a wide variety of process parameters and process conditions in view of the overall teachings contained herein, such as for example the liquid phase reaction described above. However, it is preferred in certain embodiments that this reaction step comprise a gas phase reaction, preferably in the presence of catalyst, preferably a metal catalyst, and even more preferably one or more transition metal-based catalysts (including in certain preferred embodiments transition metal halide catalysts), such as FeCl₃, chromiumoxyfluoride, Ni (including Ni mesh), NiCl₂, CrF₃, and mixture thereof, supported or in bulk Other catalysts include carbon-supported catalysts, antimony-based catalysts (such as Sb/Cl₅), aluminum-based catalyst (such as AlF₃ and Al₂O₃). It is expected that many other catalysts may be used depending on the requirements of particular embodiments, including for example palladium-based catalyst, platinum-based catalysts, rhodium-based catalysts and ruthenium-based catalysts. Of course, two or more any of these catalysts, or other catalysts not named here, may be used in combination.

In general it is preferred that the catalysts are fluorinated. In preferred embodiments, fluorination of the catalysts comprises exposing the catalyst to a stream of HF at about reaction temperature and pressure. The gas phase dehydrohalogenation reaction may be conducted, for example, by introducing a gaseous form of a compound of formula (I) into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to the desired reaction temperature.

While it is contemplated that a wide variety of reaction temperatures may be used, depending on relevant factors such as the catalyst being used and the most desired reaction product, it is generally preferred that the reaction temperature for the dehydrohalogentation step is from 150°C to 600°C, preferably 200°C to 550°C, and even more preferably from 300°C to 550°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum.

In certain embodiments, an inert diluent gas, such as nitrogen, may be used in combination with the compound of formula (I). When such a diluent is used, it is generally preferred that the compound of formula (I) comprise from 5 to greater than 95% by weight based on the combined weight of diluent and formula (I) compound.

It is contemplated that the amount of catalyst use will vary depending on the particular parameters present in each embodiment. In preferred embodiments, the contact time, which is expressed as the ratio of the volume of the catalyst (ml) to the total feed flow (ml/sec) is from 0.1 seconds to 1000 seconds, and preferably from 2 seconds to 120 seconds.

One preferred dehydrohalogenation reaction comprises a dehydrofluorination reaction. For example, for embodiments in which the desired product of formula (II) is HFO-1234yf, it is preferred that the compound of formula (I) comprises 1,1,1,2,3 pentafluoropropane. Applicants have found that in such embodiments it is preferred to use as the catalyst a nickel-based catalyst, a carbon based catalyst, or a combination of these. In highly preferred embodiments the catalyst is preferably a nickel mesh catalyst or nickel on a carbon support. In such embodiments it is also generally preferred to introduce to the reactor HF gas and inert gas, such as nitrogen, in a formula (I):HF:Inert volume ratio of from 100:20:20 to 100:80:80, with a ratio of 100:40:40 being even more preferred. In addition, it is generally preferred to conduct at least a substantial portion of the reaction at a temperature of from 450°C to 600°C. In preferred aspects mbodiments, the contact time is from 0.1 seconds to 1000 seconds, and preferably from 2 seconds to 10 seconds.

Preferably in such dehydrofluorination embodiments, the conversion is at least 50%, more preferably at least 65%, and even more preferably at least 90%. Preferably, the selectivity to HFO-1234yf is at least 70%, more preferably at least 80% and more preferably at least 90%.

Preferably in such dehydrofluorination embodiments, the conversion is at least 50%, more preferably at least 65%, and even more preferably at least 90%.

In general the direction of flow of the gaseous components may is not critical, but in certain preferred embodiments the process flow is in the down direction through a bed of the catalyst. Preferably before each cycle of use, the catalyst is dried, pre-treated and activated. It may also be advantageous in certain embodiments to periodically regenerate the catalyst after prolonged use while in place in the reactor. Pre-treatment may include heating the catalyst to 250°C to 430°C. with a stream of nitrogen or other inert gas. The catalyst may then be activated by treating it with a stream of HF diluted with a large excess of nitrogen gas in order to obtain high catalyst activity. Regeneration of the catalyst may be accomplished by any means known in the art such as, for example, by passing nitrogen over the catalyst at temperatures of from 100°C to 400°C for from 8 hours to 3 days depending on the size of the reactor.

### EXAMPLES

Certain features of the present invention are illustrated by the following examples, which should not be construed as limiting the claims in any way.

### Examples 1 - 16

These examples illustrate gas phase dehydrofluorination of CF₃CHFCH₂F (HFC-245eb) to CF₃CF=CH₂ (HFO-1234yf).

A 22-inch (1/2-inch diameter) Monel tube reactor is charged with 100 cc of catalyst, as specified in Table 1 below. A flow of 20 sccm of N₂ was maintained during the reaction. The reactor temperature is brought to the temperature indicated in the table. The HFC-245eb is passed through gas-flow controllers into a preheater maintained a temperature of about 300°C. The gas stream coming out of the preheater is passed through the catalyst bed at the desired temperature over a specified period of time. An on-line GC and a GCMS are used to analyze samples taken at the reactor exit line at regular time intervals. Finally, the reactor effluent is introduced into a 20% KOH scrubber solution at about room temperature to remove acid HF formed in-situ during the reaction. The effluent from the scrubber solution is then condensed to collect the products. The desired product CF₃CF=CH₂ (HFO-1234yf) is then isolated from the mixture by distillation.

The results are shown in Table 1 below.

**Table 1: CF₃CHFCH₂F (HFC-245eb) → CF3CF=CH2 (1234yf)**

| **Example# / Catalyst** | **T, °C** | **% Conversion of 245eb** | **% Selectivity to 1234yf** | **HFC-245eb, gm/hr** |
|---|---|---|---|---|
| Example 1/ Ni-mesh | 495 | 36 | 100 | 10 |
| Example 2/ Ni-mesh | 525 | 67 | 100 | 10 |
| Example 3/ Ni-mesh | 565 | 89 | 78 | 10 |
| Example 4/ Ni on carbon | 495 | 63 | 94 | 10 |
| Example 5/ Ni on carbon | 525 | 79 | 84 | 10 |
| Example 6/ Ni on carbon | 565 | 100 | 69 | 8 |
| Example 7/ Chromium Oxoxyfluoride | 420 | 69 | 47 | 11 |
| Example 8/ Chromium Oxoxyfluoride | 440 | 78 | 43 | 10 |
| Example 9/ Carbon | 500 | 32 | 96 | 10 |
| Example 10/ Carbon | 550 | 69 | 86 | 11 |
| Example 11/ Carbon | 600 | 85 | 76 | 12 |
| Example 12/ Pd/Carbon | 450 | 56 | 58 | 5 |
| Example 13/ Pd/Carbon | 475 | 68 | 53 | 7 |
| Example 15 4-6wt%FeCl3/C | 250 | 42 | 49 | 8 |
| Example 16 4-6wt%FeCl3/C | 300 | 59 | 37 | 8 |

### Example 17

This example illustrate liquid phase dehydrofluorination of CF₃CHFCH₂F (HFC-245eb) to CF₃CF=CH₂ (HFO-1234yf). The compound of formula (I) CF₃CHFCH₂F is stirred with 20% KOH solution in the presence or absence of 18-Crown ether at 50°C to synthesize CF₃CF=CH₂. A cleaned and leak tested 2 gallon autoclave is evacuated and then 2.5 L KOH water solution is charged into it. The KOH solution was cooled down to 0°C by a chiller. The autoclave is evacuated again and, using vacuum, 1.32Kg CF₃CFHCFH₂ is then charged to it. The sealed reactor is gradually heated with stirring to 55°C and then is heated by setting temperature at 55°C. After about 45 minutes reaction, the temperature increases to about 70°C by exothermic reaction (pressure is 11.38 bar (165 psig)). A cooling liquid is then applied to the reactor bring the temperature down to 57°C. Then the reaction is continued at 55°C for about 20 hours, and then the reaction mixture was cooled down to about 30°C and the gas product was transferred into a cylinder at dry ice-acetone temperature. About 1.1 Kg of CF₃CF=CH₂ with GC purity of about 98.6% was collected.

### Example 18

This example illustrates the addition of F₂ to CF₃CH=CH₂ in a liquid phase reaction. About 5 *-* 100 wt% F₂ in nitrogen is bubbled through 125 g of liquid trifluoropropylene (TFP) in a stirred Hastrelloy C reactor at about -20°C to about -55°C for about 1 hour in the presence of HF as the solvent. A 1 gallon Parr reactor is first charged with a relatively inert solvent, HF, to help with heat transfer and dilution of the organic. Then 125 grams of TFP is added batch wise to the reactor. The reaction mixture is continuously mixed and cooled to the desired temperature. Then the F₂ feed (10wt%), diluted with N₂ (90 wt%), is introduced continuously directly into the reaction mixture through a dip tube until about 90% of the stoiciometric amount needed to convert all the TFP that is added. The reactor temperature and pressure are controlled automatically at the desired set points of between -20 to -55°C and a constant pressure of 2.76 bar (40 psig). The temperatures are chosen to minimize the amount of TFP that would exit the reactor with the N₂ diluent. The gases exiting the reactor are passed through a caustic scrubber carboy and an activated alumina column to remove acidity, then a dri-rite column to remove moisture, and finally the organic is collected in a DIT. When the desired amount of F₂ is added the reaction liquid is sampled. The sample is absorbed in H₂O and the organic is recovered by phase separation. The organic is then analyzed by GC and GC/MS. The remaining material in the reactor is boiled off through the scrubbing system and the organic is collected in the DIT and analyzed by GC and GC/MS. The analyses are used to determine that the reaction has an overall selectivity to CF₃CHFCH₂F of about 36-45%.

### Example 19

This example illustrates addition of F₂ to CF₃CH=CH₂ in a gas phase reaction, which is illustrated by the following reaction scheme:

CF₃CH=CH₂ + F₂ → CF₃CHFCH₂F

The same apparatus as described in Example 25 is used, except that gaseous TFP and 10% F₂ (90% dilution w/ N₂) are fed into the Parr reactor via a common dip tube. TFP is fed at a 50% stoichiometric excess. The reactor is kept at -18°C and at atmospheric pressure. The reactor effluent is passed through a D1T, which collected most of the organic. Only a couple of grams of vapor are left in the Parr reactor at the end of the experiment. GC analysis of the material indicated about 10% conversion of the propylene. The selectivity to CF₃CHFCH₂F is about 52% based on GC area percentage.

## Claims

1. A method for the preparation of CF₃CF=CH₂ (HFO-1234yf), said method comprising the steps of:
a) converting CF₃CH=CH₂ to CF₃CHFCH₂F (HFC-245eb) by halogen addition, wherein the halogen addition step comprises contacting CF₃CH=CH₂ with fluorine gas; and
b) dehydrohalogenating CF₃CHFCH₂F (HFC-245eb) to form CF₃CF=CH₂ (HFO-1234yf).

2. A method according to claim 1, wherein the halogen addition reaction is a gas/liquid phase reaction.

3. A method according to claim 1, wherein the halogen addition reaction is a gas phase reaction.

4. A method according to any preceding claim, wherein the dehydrohalogenation reaction comprises exposing CF₃CHFCH₂F (HFC-245eb) to a dehydrohalogenation agent comprising potassium hydroxide (KOH).

5. A method according to claim 4, wherein the KOH is provided as an aqueous solution comprising from 10% to 50% by weight KOH.

6. A method according to claim 4 or 5, wherein the CF₃CHFCH₂F (HFC-245eb) is present in an amount from 0.9 to 10 mole %.

7. A method according to any one of claims 1-3, wherein the dehydrohalogenation reaction comprises contacting CF₃CHFCH₂F (HFC-245eb) with a nickel-based catalyst, a carbon-based catalyst or a combination thereof.

8. A method according to claim 7, wherein the dehydrohalogenation reaction is performed in the presence of hydrogen fluoride (HF) and an inert gas in a CF₃CHFCH₂F : HF : Inert gas volume ratio of from 100:20:20 to 100:80:80.

9. A method according to claim 7 or 8, wherein the dehydrohalogenation reaction is conducted at temperature of from 450°C to 600°C.

## Patentansprüche

1. Verfahren zur Herstellung von CF₃CF=CH₂ (HFO-1234yf), bei dem man:
a) CF₃CH=CH₂ durch Halogenaddition in CF₃CHFCH₂F (H-FKW 245eb) umwandelt, wobei man bei der Halogenaddition CF₃CH=CH₂ mit Fluorgas in Berührung bringt; und
b) CF₃CHFCH₂F (H-FKW 245eb) zu CF₃CF=CH₂ (HFO-1234yf) dehydrohalogeniert.

2. Verfahren nach Anspruch 1, bei dem es sich bei der Halogenaddtionsreaktion um eine Gas-/Flüssigphasenreaktion handelt.

3. Verfahren nach Anspruch 1, bei dem es sich bei der Halogenaddtionsreaktion um eine Gasphasenreaktion handelt.

4. Verfahren nach einem der vorhergehenden Ansprüche, bei dem man bei der Dehydrohalogenierungsreaktion CF₃CHFCH₂F (H-FKW 245eb) einem Kaliumhydroxid (KOH) umfassenden Dehydrohalogenierungsmittel aussetzt.

5. Verfahren nach Anspruch 4, bei dem man das KOH als wässrige Lösung mit 10 bis 50 Gew.-% KOH bereitstellt.

6. Verfahren nach Anspruch 4 oder 5, bei dem das CF₃CHFCH₂F (H-FKW 245eb) in einer Menge von 0,9 bis 10 Mol-% vorliegt.

7. Verfahren nach einem der Ansprüche 1-3, bei dem man bei der Dehydrohalogenierungsreaktion CF₃CHFCH₂F (H-FKW 245eb) mit einem auf Nickel basierenden Katalysator, einem auf Kohlenstoff basierenden Katalysator oder einer Kombination davon in Berührung bringt.

8. Verfahren nach Anspruch 7, bei dem man die Dehydrohalogenierungsreaktion in Gegenwart von Fluorwasserstoff (HF) une eines Inertgases in einem CF₃CHFCH₂F:HF:Inertgas-Volumenverhältnis von 100:20:20 bis 100:80:80 durchführt.

9. Verfahren nach Anspruch 7 oder 8, bei dem man die Dehydrohalogenierungsreaktion bei einer Temperatur von 450°C bis 600°C durchführt.

## Revendications

1. Procédé de préparation de CF₃CF = CH₂ (HFO-1234yf), ledit procédé comprenant les étapes de :
a) transformation de CF₃CH=CH₂ en CF₃CHFCH₂F (HFC-245eb) par l'ajout d'halogène, l'étape d'ajout d'halogène comprenant la mise en contact de CF₃CH=CH₂ avec le fluor gazeux ; et
b) déhydrohalogénation de CF₃CHFCH₂F (HFC-245eb) pour former le CF₃CF=CH₂ (HFO-1234yf) .

2. Procédé selon la revendication 1, **caractérisé en ce que** la réaction d'ajout d'halogène est une réaction en phase gazeuse/liquide.

3. Procédé selon la revendication 1, **caractérisé en ce que** la réaction d'ajout d'halogène est une réaction en phase gazeuse.

4. Procédé selon l'une quelconque des revendications précédentes, **caractérisé en ce que** la réaction de déhydrohalogénation comprend l'exposition de CF₃CHFCH₂F (HFC-245eb) à un agent de déhydrohalogénation comprenant l'hydroxyde de potassium (KOH).

5. Procédé selon la revendication 4, **caractérisé en ce que** le KOH est proposé en solution aqueuse comprenant de 10 % à 50 % en poids de KOH.

6. Procédé selon la revendication 4 ou 5, **caractérisé en ce que** le CF₃CHFCH₂F (HFC-245eb) est présente dans une quantité de 0,9 à 10 % en mole.

7. Procédé selon l'une quelconque des revendications 1 à 3, **caractérisé en ce que** la réaction de déhydrohalogénation comprend la mise en contact de CF₃CHFCH₂F (HFC-245eb) avec un catalyseur à base de nickel, un catalyseur à base de carbone ou une combinaison de ceux-ci.

8. Procédé selon la revendication 7, **caractérisé en ce que** la réaction de déhydrohalogénation est effectuée en présence de fluorure d'hydrogène (HF) et d'un gaz inerte dans un rapport en volume CF₃CHFCH₂F:HF:gaz inerte de 100:20:20 à 100:80:80.

9. Procédé selon la revendication 7 ou 8, **caractérisé en ce que** la réaction de déhydrohalogénation est effectuée à une température de 450°C à 600°C.
